# EUROPEAN PATENT APPLICATION

(11) **EP 4 811 365 A1**
(43) Date of publication of application: **23.09.2026**
(21) Application number: 26166324.9
(22) Date of filing: 20.03.2026
(51) Int. Cl.: G16C 20/62, G16B 35/10

(54) **METHOD FOR PRODUCING LIBRARY, LIBRARY PRODUCTION APPARATUS, PROGRAM, AND METHOD FOR SEARCHING FOR APERIODIC POLYMER**

(30) Priority: 21.03.2025 JP 2025046606
(71) Applicant: Konica Minolta, Inc., Tokyo 100-7015 (JP)
(72) Inventor: ASAI, Yuichiro, Tokyo 100-7015 (JP); INOUE, Satoru, Tokyo 100-7015 (JP); NOBA, Kosaku, Tokyo 100-7015 (JP); TAKAHASHI, Rieko, Tokyo 100-7015 (JP)
(74) Representative: MFG Patentanwälte Meyer-Wildhagen Meggle-Freund Gerhard PartG mbB

(57) **Abstract**

A method for producing a library that includes a plurality of types of aperiodic polymers, each of the aperiodic polymers being an aperiodic polymer, and the method including: generating that is generating a plurality of types of the aperiodic polymers by combining two or more types of monomers selected from a monomer group; evaluating that is evaluating, by using an evaluation function, diversity in a combination of the generated plurality of types of the aperiodic polymers; and extracting that is extracting a combination of the aperiodic polymers for which an evaluation result is equal to or greater than a threshold value. The evaluation function is a function for evaluating diversity based on two or more types of parameters.

## Description

### BACKGROUND OF THE INVENTION

### TECHNICAL FIELD

The present disclosure relates to a method for producing a library, a library production apparatus, a program, and a method for searching for an aperiodic polymer.

### DESCRIPTION OF RELATED ART

Biomolecules (biocompounds) such as nucleic acids, proteins, and peptides react with, for example, selectively bind to targets, and have various functions such as detection of targets, inhibition of functions, and purification. For this reason, bio-compounds are attracting attention for use in a wide range of areas such as diagnosis, medicine, and chemical production.

The relation between the structure and function of a bio-compound is unique, unlike a low molecular weight compound and a general high molecular weight compound. For example, a protein and a nucleic acid aptamer have a primary structure in which amino acids and nucleotides, respectively, are linked non-periodically as monomers, and these are aperiodic polymers. The aperiodic polymer can selectively and strongly bind to a target molecule by assuming an appropriate higher-order structure due to, for example, intramolecular hydrogen bonding.

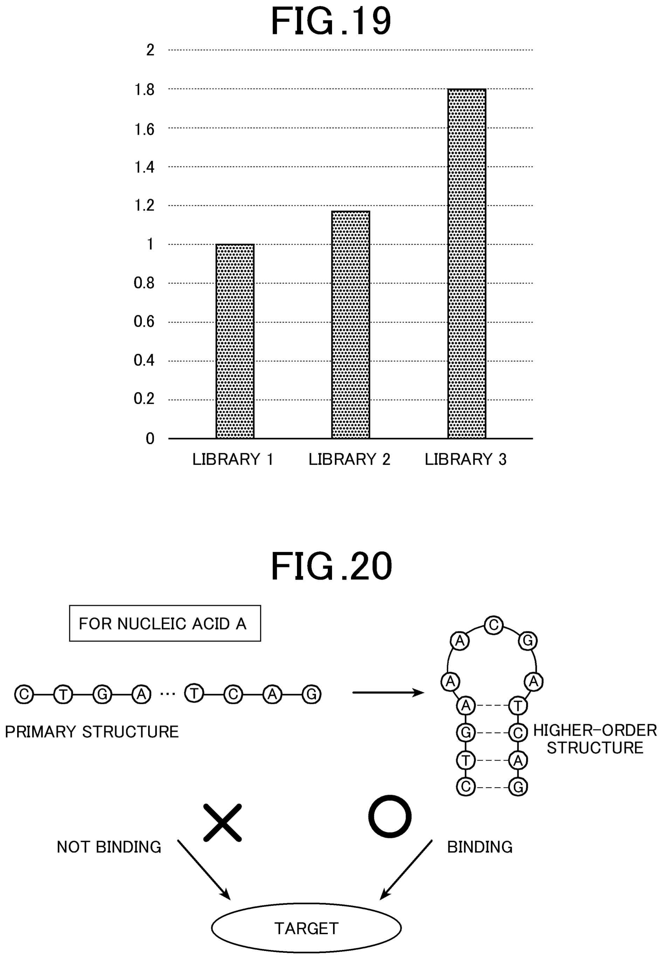

FIG. 20 is a diagram for explaining the binding action of a nucleic acid A, which is one of aperiodic polymers, to a target. The nucleic acid A does not react with the target in a state of not forming a higher-order structure, but reacts with the target by forming a higher-order structure.

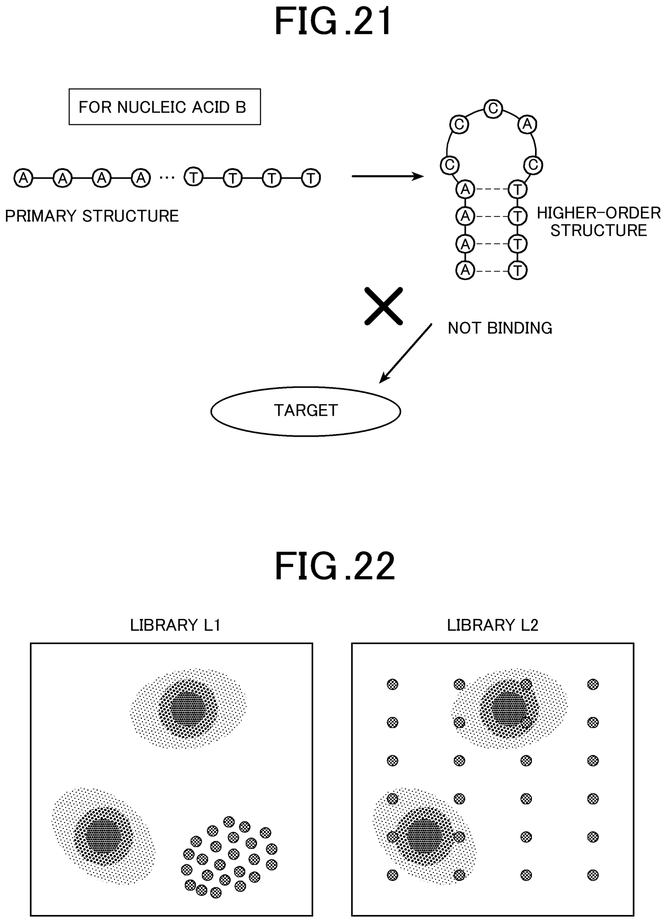

Further, even if the aperiodic polymers have the same higher-order structure, when the sequence in the primary structure is different, the reaction with the target is also different. Fig. 21 is a diagram illustrating a binding action of a nucleic acid B, which is one of aperiodic polymers, to a target. The nucleic acid A illustrated in Fig. 20 and the nucleic acid B illustrated in Fig. 21 have different primary structures. Even if the nucleic acid B has the same higher-order structure as the nucleic acid A, it does not react with the target.

That is, both the primary structure and the higher-order structure needs to be adjusted in order to obtain an aperiodic polymer that reacts with a target. In addition, since targets are diverse, diversity is also required for a library in which a plurality of types of candidates for an aperiodic polymer to react with a target are collected.

Fig. 22 is a diagram illustrating the diversity of a library. The plurality of dots represent each aperiodic polymer included in the library, and the plurality of areas of the ellipses represent spatial areas of compounds having desired properties. In the library L1, a plurality of points are concentrated in a certain area, and no point exists in the area of the ellipse. That is, there is a high possibility that the properties of the respective aperiodic polymers are similar in the library L1, the diversity is low, and a compound having a desired property does not exist. On the other hand, in the library L2, plurality of points are dispersed, and a point exists also in the area of the ellipse. That is, in the library L2, there is a high possibility that the properties of the aperiodic polymers vary, the diversity is high, and both a compound having a desired property and a compound having no desired property are present.

Here, when the desired property is a property that reacts with a target, the library L1 does not contain an aperiodic polymer that reacts with a target. Therefore, it is necessary to search for an aperiodic polymer that reacts with a target from another library, and the efficiency of searching for an aperiodic polymer that reacts with a target is low. On the other hand, since the aperiodic polymer that reacts with the target is contained in the library L2, it is not necessary to further search another library, and the efficiency of searching for the aperiodic polymer that reacts with the target is high. That is, by increasing the diversity of the library, the efficiency of searching for an aperiodic polymer that reacts with a target is improved.

As a method for searching for an aperiodic polymer that reacts with a target, the SELEX method is known (U.S. Pat. No. 5475096). In the SELEX method, first, monomers are linked one by one while adding each monomer species in an equal amount, to prepare a library of aperiodic polymers composed of random primary structures. Next, the library is brought into contact with the target, and only the aperiodic polymer that has reacted with the target is recovered, amplified by PCR (Polymerase Chain Reaction), and brought into contact with the target again, and this process is repeated.

### SUMMARY OF THE INVENTION

In the technique disclosed in U.S. Pat. No. 5475096, the primary structure of each monomer species is random, but on the other hand, since the primary structure is not selected so as to form a specific higher-order structure, the specific higher-order structure is hardly formed. As a result, since the aperiodic polymers contained in the library have a low diversity of higher-order structures, only a very small number of aperiodic polymers react with the target, and the search efficiency is low. As described above, it is not easy to construct a library of aperiodic polymers having a high diversity of plurality of parameters such as a primary structure and a higher-order structure.

An object of the present disclosure is to provide, for example, a method for producing a library of aperiodic polymers with high search efficiency.

In order to solve the above-described problem, the inventor of the present disclosure has studied the cause of the above-described problem and the like. The method for producing a library includes a generation step, an evaluation step for evaluating diversity using an evaluation function, and an extraction step for extracting an aperiodic polymer having an evaluation result equal to or higher than a threshold value. The evaluation function is a function for evaluating diversity on the basis of at least two or more types of parameters. Thus, it has been found that a library having high search efficiency can be produced, which has led to the present disclosure.

That is, the above problem according to the present disclosure is solved by the following means.

To achieve at least one of the abovementioned objects, according to an aspect of the present invention, method for producing library reflecting one aspect of the present invention is
a method for producing a library that includes a plurality of types of aperiodic polymers, each of the aperiodic polymers being an aperiodic polymer, and the method comprising:
generating that is generating a plurality of types of the aperiodic polymers by combining two or more types of monomers selected from a monomer group;
evaluating that is evaluating, by using an evaluation function, diversity in a combination of the generated plurality of types of the aperiodic polymers; and
extracting that is extracting a combination of the aperiodic polymers for which an evaluation result is equal to or greater than a threshold value, wherein
the evaluation function is a function for evaluating diversity based on two or more types of parameters.

### BRIEF DESCRIPTION OF THE DRAWINGS

The advantages and features provided by one or more embodiments of the invention will become more fully understood from the detailed description given hereinbelow and the appended drawings which are given by way of illustration only, and thus are not intended as a definition of the limits of the present invention, wherein:
Fig. 1 is a flowchart illustrating a method of searching for and collecting an aperiodic polymer that reacts with a target, using a library;
Fig. 2 is a diagram illustrating the same library comprising aperiodic polymers reacting on different targets A and B;
Fig. 3 is a diagram illustrating the same library containing aperiodic polymers reactive against different targets A and B;
Fig. 4 is a diagram illustrating the same library containing aperiodic polymers reactive against different targets A and B;
Fig. 5 is a diagram illustrating the same library comprising aperiodic polymers reacting on different targets A and B;
Fig. 6 is a flowchart illustrating a method for producing a library;
Fig. 7 is an example of a type of higher-order structure;
Fig. 8 is a diagram illustrating a method of reconnecting disassembled modules in different combinations;
Fig. 9 is a diagram illustrating the configurations of polymers in polymer populations G1 and G2 when the horizontal axis represents a parameter type P1 and the vertical axis represents a parameter type P2 different from P1;
Fig. 10 is a block diagram illustrating a schematic configuration of a library production apparatus;
Fig. 11 is a histogram in example 1;
Fig. 12 is a graph representing calculation value of primary structure parameters;
Fig. 13 is a graph representing calculation values of higher-order structure parameters;
Fig. 14 is a graph representing the sum of the calculation value of a primary structure parameter and the calculation value of a higher-order structure parameter;
Fig. 15 is a graph illustrating the maximum intensity of fluorescence;
Fig. 16 is a histogram in example 3;
Fig. 17 is a graph representing calculation values of primary structure parameters;
Fig. 18 is a graph representing calculation values of higher-order structure parameters;
Fig. 19 is a graph representing the sum of the calculation value of the primary structure parameter and the calculation value of the higher-order structure parameter;
Fig. 20 is a diagram illustrating the binding action of nucleic acid A, which is one of the aperiodic polymers, to a target;
Fig. 21 is a diagram illustrating the binding action of nucleic acid B, which is one of the aperiodic polymers, to a target; and
Fig. 22 is a diagram illustrating the diversity of a library.

### DETAILED DESCRIPTION

Hereinafter, some embodiments according to the present disclosure will be described with reference to the drawings. However, the scope of the invention is not limited to the disclosed embodiments.

The method for producing a library of the present disclosure produces a library including a plurality of types of aperiodic polymers. The method for producing a library includes a generation step, an evaluation step, and an extraction step. The generating step generates a plurality of types of aperiodic polymers by combining at least two or more types of monomers selected from the group of monomers (monomer group). The evaluation step evaluates the diversity of combinations of the generated plurality of types of aperiodic polymers using an evaluation function. In the extraction step, a combination of aperiodic polymers whose evaluation result is equal to or more than a threshold value is extracted. The evaluation function is a function for evaluating diversity on the basis of at least two or more types of parameters. The above is the characteristic.

The above features are technical features common to or corresponding to the following embodiments.

In the present embodiment, the parameters preferably include at least two or more types selected from the group consisting of primary structure, higher-order structure, type of element, number of atoms, coordinate of atoms, position of atoms, free energy of the most stable structure, proportion of the most stable structure, melting temperature, length, polarizability, polarity, reactivity, orientation, solubility, degree of similarity between aperiodic polymers, predictions thereof and conversions thereof in aperiodic polymers. Thus, the diversity of the library is improved, and the search efficiency can be improved.

In the present embodiment, it is preferable that the parameter includes at least one or more types belonging to a group including a primary structure, a higher-order structure, a predicted primary structure, a converted primary structure, a predicted higher-order structure, and a converted higher-order structure. Thereby, the diversity of the structure of the library is improved, and the search efficiency can be improved.

In the present embodiment, the primary structure is preferably generated in the generation step so that the aperiodic polymer has a predetermined higher-order structure. As a result, the diversity of the higher-order structure of the library is improved, and the search efficiency can be improved.

In the present embodiment, the primary structure is preferably generated in the generation step so that the aperiodic polymer is most stable in terms of energy when the aperiodic polymer has a predetermined higher-order structure. As a result, the diversity of the higher-order structure of the library is improved, and the search efficiency can be improved.

In the present embodiment, it is preferable to repeat the evaluation step and the extraction step a plurality of times. Thus, the calculation process can be simplified, and the search efficiency can be improved.

In the present embodiment, the number of the monomers constituting one molecule of the aperiodic polymer is preferably within a range of 10 to 100. Thus, the aperiodic polymer can have a higher-order structure. In addition, the number of types of the aperiodic polymer does not become too large, the coverage of the library can be secured, and the search efficiency can be improved.

In the present embodiment, the aperiodic polymer is preferably a nucleic acid. Thus, a nucleic acid that reacts with the target can be searched for in the library.

The library production apparatus of the present disclosure produces a library containing a plurality of types of aperiodic polymers. The library production apparatus includes a generation section, an evaluation section, and an extraction section. The generation section generates a plurality of types of aperiodic polymers by combining at least two or more types of monomers selected from a monomer group. The evaluation section evaluates diversity in a combination of the generated plurality of types of aperiodic polymers by using an evaluation function. The extraction section extracts a combination of aperiodic polymers whose evaluation result is equal to or more than a threshold value. The evaluation function is a function for evaluating diversity on the basis of at least two or more types of parameters.

A program of the present disclosure causes a computer in a library production apparatus for producing a library containing a plurality of types of aperiodic polymers to execute a generation process, an evaluation process, and an extraction process. In the generation process, at least two or more types of monomers selected from the monomer group are combined to produce a plurality of types of aperiodic polymers. The evaluation processing evaluates the diversity in the combination of the generated plurality of types of aperiodic polymers using an evaluation function. In the extraction process, a combination of aperiodic polymers having an evaluation result equal to or greater than a threshold value is extracted. The evaluation function is a function for evaluating diversity on the basis of at least two or more types of parameters.

The method for searching for an aperiodic polymer of the present disclosure searches for an aperiodic polymer that reacts with a target, using a library. A method for searching for an aperiodic polymer includes a step of producing a library using the above-described production method, a step of contacting the produced library with a target, and a step of collecting the aperiodic polymer that has reacted with the target.

The library is desirably reacted with a plurality of targets. Thus, it is possible to search for an aperiodic polymer that exhibits a selective reaction, such as reacting with the target A but not reacting with the target B, or reacting with both the target A and the target B.

Hereinafter, one or more embodiments of the present disclosure will be described with reference to the drawings. However, the scope of the present disclosure is not limited to the disclosed embodiment. In the present description, when two numbers are used to indicate a range of value before and after "to", these numbers are included in the range as the lower limit value and the upper limit value.

### 1 Library

In the present embodiment, the term "library" refers to a collection of aperiodic polymers as candidate, and the library is used to search for an aperiodic polymer that reacts with a target. The types of the aperiodic polymers contained in the library are not particularly limited, and are preferably in the range of 10 types to 1 billion types, and more preferably in the range of 100 types to 10 million types. Note that in the present embodiment, the library may be a group of aperiodic polymers virtually generated on a computer, or may be a group of aperiodic polymers virtually generated on a computer and then actually synthesized in a laboratory or the like.

The type of the target is not particularly limited. The target may be, for example, a compound or the like used in the medical field or a compound or the like used in the industrial field. The target may be a high molecular weight compound or a low molecular weight compound. Furthermore, the target may be a biopolymer such as a protein. The target may be a complex in which a plurality of proteins are associated, and may be a cell, a virus, or the like. The target may be an aggregation or a crystal. The target may be inorganic or may be an inorganic complex or ion.

The target is comprised, for example, in a biological sample. Biological samples include whole blood, leukocytes, peripheral blood mononuclear cells, plasma, serum, sputum, breath, urine, semen, saliva, meningeal fluid, amniotic fluid, glandular fluid, lymphatic fluid, nipple aspirate, bronchial aspirate, synovial fluid, joint aspirate, cells, cell culture, cell extracts, stool, tissue extracts, cerebrospinal fluid and the like.

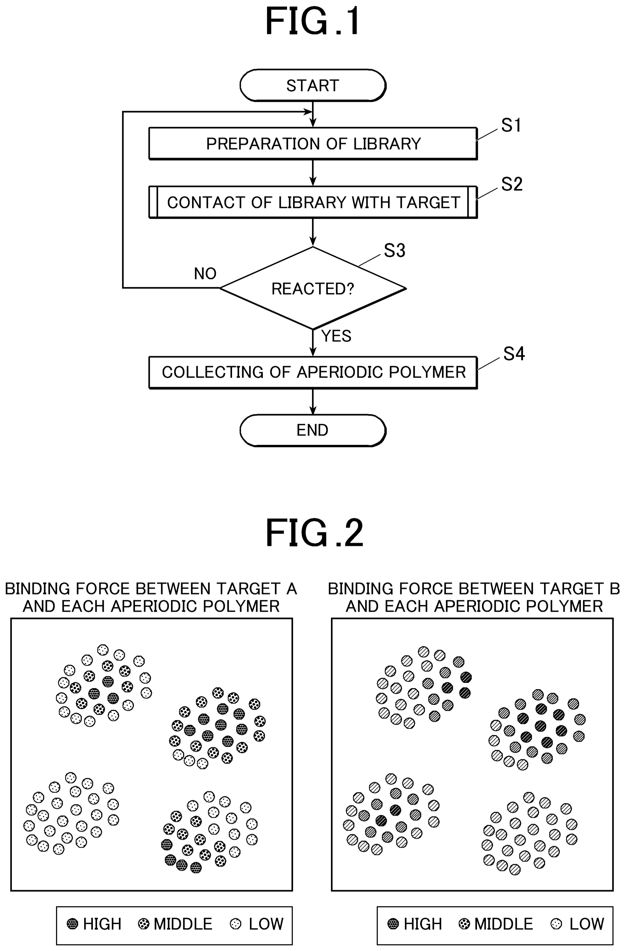

FIG. 1 is a flowchart illustrating a method of searching for and collecting an aperiodic polymer that reacts with a target using a library. First, a library is prepared in step S1. Next, in step S2, the prepared library is brought into contact with a target. Next, in step S3, the presence or absence of the aperiodic polymer that has reacted with the target is confirmed. When the aperiodic polymer reacted with the target can be confirmed (S3:YES), the process proceeds to step S4. If an aperiodic polymer that reacted with the target cannot be confirmed (S3:NO), the steps S1 to S3 are repeated again because the library used does not contain an aperiodic polymer that reacts with the target. In step S4, the aperiodic polymer that has reacted with the target is collected. Note that the steps S2 to S4 are not particularly limited, and known methods may be used.

Since the search performance can be improved by reducing the number of iterations of steps S1 to S3, it is preferable that the library contains a large number of aperiodic polymers having variations in properties, that is, has diversity. In addition, even in the case of searching for one or more types of aperiodic polymers that react with a plurality of types of targets, the same library can be used as long as the library has diversity. Thus, the step S1 of creating a library can be omitted, and the search performance can be improved.

Fig. 2 to Fig. 5 are diagrams illustrating the same library including aperiodic polymers that react with different targets A and B. The right side of Figs. 2 to 5 shows the reactivity with the target A, and the left side of Figs. 2 to 5 shows the reactivity with the target B. The libraries in FIGS. 2-5 are all identical, and each point represents the degree (high, medium, low) in reactivity of each aperiodic polymer.

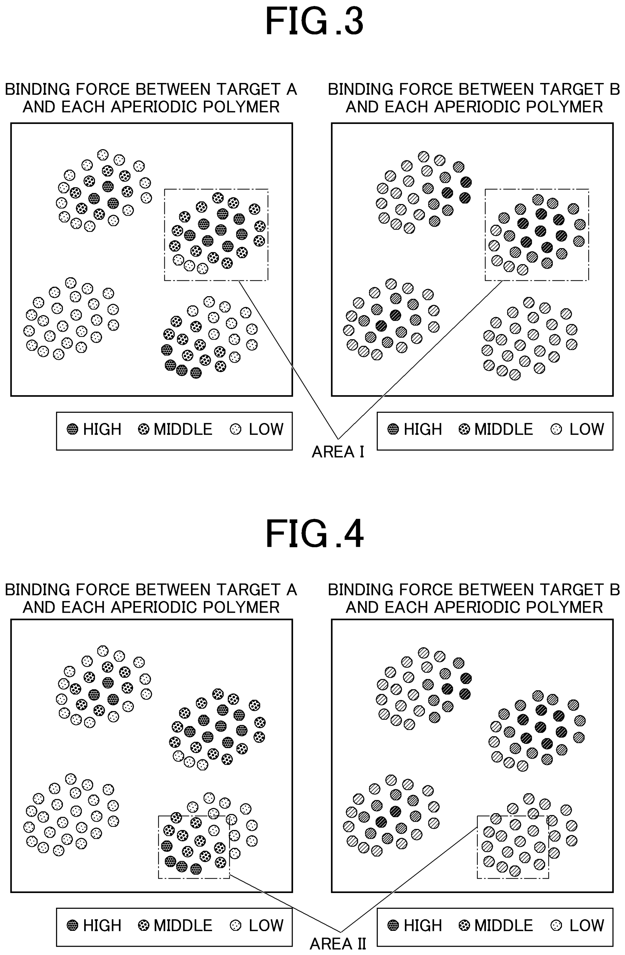

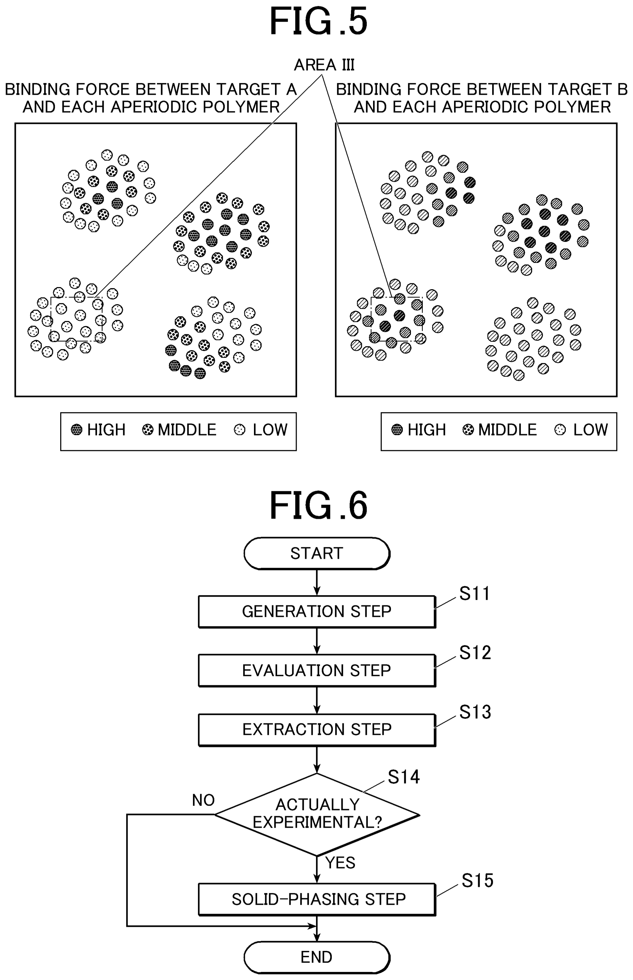

As illustrated in Fig. 3, since the polymer reacts with both the targets A and B in the area I, an aperiodic polymer that reacts with both the targets A and B can be searched for. As illustrated in Fig. 4, since the polymer reacts only with the target A in the area II, an aperiodic polymer that reacts only with the target A can be searched for. As illustrated in Fig. 5, since the polymer reacts only with the target B in the area III, an aperiodic polymer that reacts only with the target B can be searched for. That is, it can be seen that an aperiodic polymer that reacts with both targets A and B, an aperiodic polymer that reacts only with target A, and an aperiodic polymer that reacts only with target B can be searched for using the same library.

When the library prepared in step S1 is an aggregate of aperiodic polymers virtually generated on a computer, the subsequent steps S2 to S4 can also be virtually executed on the computer. In addition, when the library prepared in the step S1 is an aggregate of aperiodic polymers actually synthesized in a laboratory or the like after being virtually generated on a computer, the subsequent steps S2 to S4 can also be actually performed in the laboratory, and the actual aperiodic polymer reacted with the target can be finally recovered.

### 2. method for producing library

Fig. 6 is a flowchart illustrating a method of producing a library of the present embodiment. First, in the production step S11, at least two or more types of monomers selected from the monomer group are combined to produce a plurality of types of aperiodic polymers. Next, in the evaluation step S12, the diversity in the combination of the plurality of types of produced aperiodic polymers is evaluated using an evaluation function. Next, in the extraction step S13, a combination of aperiodic polymers having an evaluation result equal to or greater than a threshold value is extracted. Thereafter, in the determination step S14, it is determined whether or not the prepared library is actually brought into contact with a target for an experiment. In the case of an actual experiment (S14:YES), in the immobilization step S15, the extracted aperiodic polymers are subjected to solid-phase synthesis, or each of the extracted aperiodic polymers is synthesized, and a combination of the synthesized aperiodic polymers is immobilized to obtain a library. When the experiment is not actually performed but is virtually performed (S14:NO), combinations of the extracted aperiodic polymers are used as a library.

### (1) Generation step

### (1. 1) aperiodic polymer

In the present embodiment, the term "aperiodic polymer" refers to a polymer having a primary structure obtained by combining and connecting at least two or more types of monomers selected from the group of monomers in an aperiodic manner.

When the number of types of monomers contained in the monomer group is represented by n and the number of monomers to be combined is represented by m, the number of combinations of primary structures of the aperiodic polymer can be represented by n^{m}. The greater the number of combinations n^{m}, the greater the diversity of the library.

The number n of types of monomers is not particularly limited, but is preferably four or more. When the number n of types is 4 or more, the library contains a large number of aperiodic polymers having variations in properties and has diversity. Thus, in the above step S2 and S3, time, effort, and the like required for operation can be reduced, and search efficiency can be improved. The number m of the monomers is not particularly limited, but is preferably within a range of 10 to 100. All of the m monomers may be of different types, or some of them may be of the same type. When the number is 10 or more, the aperiodic polymer can have a higher-order structure. In addition, when the number is 100 or less, the number of combinations of the monomers does not become too large, and the search efficiency can be improved.

In the present embodiment, the arrangement of the monomers in the aperiodic polymer is referred to as a "primary structure". In addition, a secondary or higher structure of the aperiodic polymer, for example, a secondary structure, a tertiary structure, and a quaternary structure are collectively referred to as a "higher-order structure". What kind of higher-order structure the aperiodic polymer can have depends on the primary structure. In the present embodiment, the "structure" includes a predicted structure and a converted structure.

Specific examples of the aperiodic polymer include a protein, a peptide, and a nucleic acid, and among these, a nucleic acid is preferable. When actually synthesized in the laboratory, the nucleic acid can be synthesized on an object. For example, it can be synthesized on a bead-like object made of glass, silica, or polyester, or can be synthesized on a substrate by using an inkjet technique or a photolithography technique. In the case of a bead-like object, it is possible and convenient to synthesize a different aperiodic polymer for each bead. In the case of synthesis on a substrate, for example, a specific aperiodic polymer can be synthesized while designating the position, and for example, a large number of aperiodic polymers can be synthesized in a state in which the respective coordinates are clarified. Therefore, when a target is brought into contact and optically visualized, the reaction between the target and any aperiodic polymer can be measured, which is very useful in searching for a aperiodic polymer that reacts with the target. The types of the monomers include, for example, those constituting nucleic acids, proteins, and peptides, but the form thereof is not particularly limited as long as the monomers can be linked and synthesized, and the monomers are not limited to naturally occurring monomers.

The aperiodic polymer may be modified with a low molecular weight functional group at the terminal thereof. Examples of the low molecular weight functional group include biotin and an amino group. In the present embodiment, a low molecular weight functional group for modifying the terminal is also included in the monomer. That is, the monomer includes a low molecular weight functional group having one or more functional groups in addition to the monomer having two or more polymerizable functional groups. In addition, a linker or a spacer may be appropriately provided depending on the purpose such as modification efficiency or securement of reactivity with a target.

The aperiodic polymer may include an artificially created polymer. Examples of the artificially created polymer include a modified nucleic acid, an artificial nucleic acid, an artificial amino acid, and a luminescent color.

Nucleic acids include naturally occurring nucleic acids, modified nucleic acids and artificial nucleic acids. Naturally occurring nucleic acids include:
- DNA(Deoxyribonucleic acid)
- RNA(Ribonucleic acid)

Examples of modified nucleic acids and artificial nucleic acids include the following.
- LNA(locked nucleic acid)
- BNA(bridged nucleic acid)
- PNA(peptide nucleic acid)
- Phosphorothioate
- 2'-OMe, amido-bridged nucleic acid (AmNA)
- Phosphorylation modification • FAM (fluorescein)
- Alexa Fluor(Registered trade mark, manufactured by Thermo Fisher Scientific
- Cy3 ^{®} (manufactured by GE Healthcare Co., Ltd)
- Cy5 ^{®} (manufactured by GE Healthcare Co., Ltd)
- TAMRA
- Rhodamine
- BHQ ^{®} (manufactured by LGC Biosearch Technologies Co., Ltd)
- Dabyl
- 2-aminopurine
- 5-Bromo dU
- Deoxiuridine
- Biotin
- Streptavidin
- Ethynyl dU

### (1. 2) Generation Method

Methods of producing aperiodic polymers are described. The method of generating the aperiodic polymer is not particularly limited, but a method of using any calculator is preferable from the viewpoint that a large amount of the aperiodic polymer can be simply generated. For example, a physical computer may be used, or a virtual environment such as a server may be used.

In the production of an aperiodic polymer, once a primary structure is selected, a higher-order structure is determined based on the selected primary structure. However, in actual experiments, when a primary structure is selected, a higher-order structure is not necessarily determined uniquely, and a plurality of types of higher-order structures may be formed depending on the type and sequence of monomers in the primary structure. Therefore, it is preferable to select a primary structure such that a higher-order structure is unambiguously determined.

From the viewpoint of enhancing diversity, the primary structure of the monomer has aperiodicity. A method of combining the monomers is not particularly limited.

The primary structure of the monomer can be represented as follows.

For example, an example in which a plurality of aperiodic polymers are produced from five types of monomers, i.e., adenine as A, thymine as T, cytosine as C, guanine as G, and 2-aminoadenosine as Z, is shown below. Note that the use of letters such as the alphabet provides high visibility.
ATGCZTATCGGGCGA
AAAAATCGGGGGCGGGGGAAAAAAAACCAAAA
TTCGGGGCGAGAGTGACZZZGAGAGT

Numerals may be used instead of alphabets. In particular, a notation using a matrix or a notation that is easily converted into a matrix is advantageous in the case of performing matrix calculation, for example, in the case of using machine learning. For example, any method such as encoding, one hot encoding, and embedding can be used. For example, an example in which a plurality of aperiodic polymers are produced from four types of monomers, i.e., 1 for adenine, 2 for thymine, 3 for cytosine, and 4 for guanine, is shown below. Note that in the following example, the gaps are filled with 0 s to equalize the lengths of the aperiodic polymers.
11231232324432321231221123000000
42122233212342224231110000000000

The primary structure may have at least aperiodicity, but in addition, it is preferable to select the primary structure so that the aperiodic polymer has a predetermined higher-order structure.

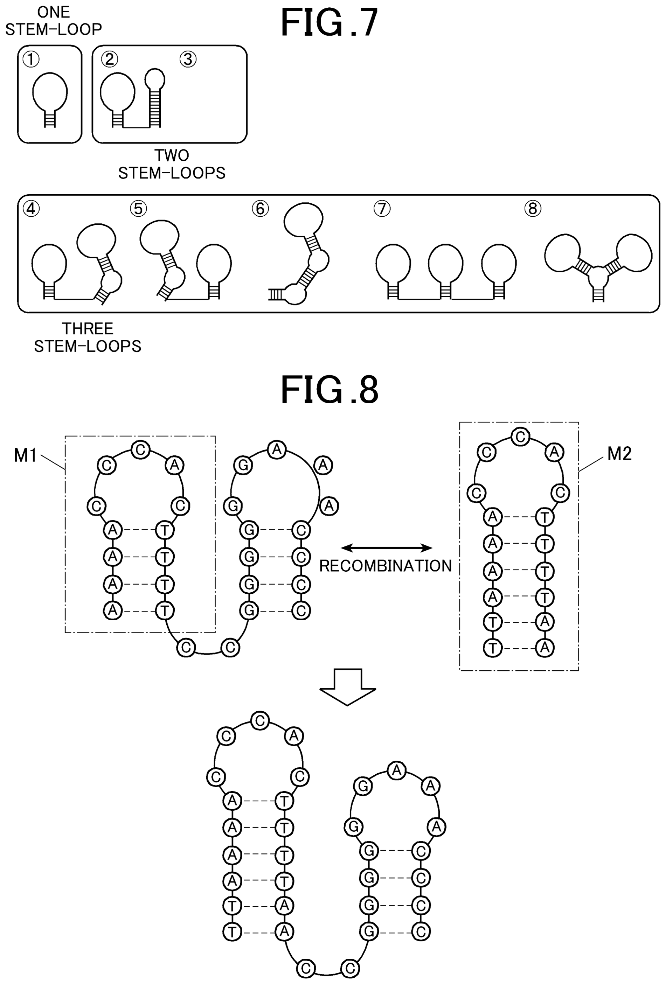

Fig. 7 is an example of a type of a higher-order structure. The aperiodic polymer aggregate can have increased diversity by containing different types of higher-order structures. For example, it is preferable to select the primary structure so that each of the eight types of higher-order structures shown in FIG. 7 can be generated with high probability.

In the selection of the primary structure, an algorithm, machine learning, or a rule focusing on the properties of an aperiodic polymer may be used. Examples of the algorithm, machine learning, or rule include sorting, K-means, the K-nearest neighbor method, the EM algorithm, Gaussian process regression, logistic regression, support vector machine (SVM), simple regression, multiple regression, Lasso regression, Ridge regression, elastic net, sparse model, support vector regression (SVR), decision tree, random forest, neural net, deep learning model, and function. Examples of the deep learning model include a variational autoencoder (VAE) model, a generative adversarial network (GAN), a flow-base generative model (FLOW), and a diffusion model. These may be used in combination. By using these algorithms, machine learning, or rules, a primary structure can be selected while reflecting a specific intention.

For example, an RNA having a function of mimicking a RNA family can be generated by allowing the VAE model to learn RNA family observed in a living body.

Using genetic algorithm, plurality of aperiodic polymers may be multiplied to produce various aperiodic polymers. Examples of the method of multiplying include a method of patching structures, and a method of converting an aperiodic polymer into a vector and rearranging its elements.

In addition, the higher-order structure of the aperiodic polymer may be focused.

The higher-order structure may be decomposed into modules, and the decomposed modules may be bound again in different combinations.

Fig. 8 is a diagram illustrating a method of reconnecting disassembled modules in a different combination. In the example of Fig. 8, in the aperiodic polymer including the module M1, the length of the double helix structures can be extended by replacing the module M1 with the module M2. Note that the term "module" herein refers to a structural unit determined by a higher-order structure, and many of "modules" are also characteristic or functional units.

When the selected tentative primary structure does not take the predetermined higher-order structure, the tentative primary structure may be mutated so as to take the predetermined higher-order structure. In this case, the free energy is calculated on the assumption that the primary structure to which the mutation is added has a predetermined higher-order structure, and the primary structure in which the predetermined higher-order structure is most stable in terms of energy is selected. By using this method, a primary structure that forms a predetermined higher-order structure with a higher probability can be selected. Furthermore, since an area to which a mutation can be added and an area to which a mutation is not added can be specified with respect to the tentative primary structure, important characteristics can be retained not only with respect to the higher-order structure but also with respect to the primary structure, and the diversity of the primary structure can also be retained.

### (2) Evaluation step

Among the aperiodic polymers produced in the production step S11, a plurality of types are selected to form a combination of aperiodic polymers (polymer population). Furthermore, the combination is changed to form a plurality of polymer populations. For each of the formed polymer populations, diversity is evaluated using an evaluation function.

The greater the number of aperiodic polymers forming a polymer population, the greater the diversity of the polymer population. On the other hand, the smaller the number of aperiodic polymers forming the polymer population, the smaller the number of aperiodic polymers in the finally obtained library. Thereafter, when the library is used to search for an aperiodic polymer that reacts with a target, the calculation step can be simplified and the search efficiency is increased. Therefore, it is preferable that the polymer population is formed of a relatively small number of aperiodic polymers while having sufficient diversity. One million aperiodic polymers can be synthesized on one microarray, or 6,000,000 aperiodic polymers in the case of using a semiconductor, and these can be compared at the same time in one experiment. Furthermore, when a plurality of these are used at the same time, many times as many aperiodic polymers can be experimentally evaluated at the same time. From such a viewpoint, the number of the aperiodic polymers forming the polymer population is not limited, but is preferably in the range of 10 to 1 billion types, and more preferably in the range of 100 to 10 million.

### (parameter)

The evaluation function is a function for evaluating diversity on the basis of at least two or more types of parameters. The polymer population preferably covers a broad range for a predetermined parameter. In addition, it can be said that as the number of parameter types covering such a wide range is larger or as a proportion covered by parameters is higher, there is diversity.

The parameters are not particularly limited as long as they are variables that characterize the structure, properties, function, and the like of the aperiodic polymer. For example, as the parameter, a variable for characterizing a structural property or a physicochemical property of the aperiodic polymer is useful. Furthermore, when a function of the aperiodic polymer inside and outside a living body has been reported, a variable that characterizes this function is useful as a parameter.

Examples of the physicochemical parameters include polarizability, polarity, molecular weight, isoelectric point pI, melting temperature Tm, Gauss free energy of the most stable structure, length, viscosity, light absorption characteristics, direction (orientation characteristics), and solubility. Examples of the structural parameters include a primary structure, a higher-order structure, the type of an element, the number of atoms, the coordinate of atoms, the position of an atom, the proportion of assuming a specific higher-order structure in a solution, the proportion of assuming an arbitrary higher-order structure in a solution, and the like. Note that the primary structure as a parameter herein includes the types of monomers, the number (proportion) of each monomer species, and the like. For example, in the case of deoxyribonucleic acid (DNA), the primary structure as a parameter also includes the number (proportion) of adenine, thymine, guanine, and cytosine. The higher-order structure as a parameter here also includes the angle of twist of the double helix structure, the length of the double helix structure, the length of the loop structure, the number of loop structures, and the like. Furthermore, the parameters include ones obtained by converting one example of these parameters by a machine learning model, a rule, a function, an algorithm, or the like.

These parameters may be converted into different forms. For example, the primary structure and the higher-order structure may be converted into vector or the like. From the standpoint of ease of calculation of the evaluation function, the parameters are preferably converted into a mathematical form, more preferably into the form of, for example, numerical values, a vector, a matrix, or the like. The form of the parameter may be an actually measured value, a calculation value, or a predicted value.

In addition, a parameter that characterizes the aperiodic polymers may be separately calculated from these parameters. Examples of the parameter for characterizing the aperiodic polymers include the similarity between the aperiodic polymers. In the present embodiment, the "similarity between aperiodic polymers" is a degree representing the similarity of features between aperiodic polymers, and the "similarity" also includes a "distance". The term "distance" as used herein refers to a degree indicating how far features in aperiodic polymers are separated from each other, that is, how low the similarity is.

### (evaluation function)

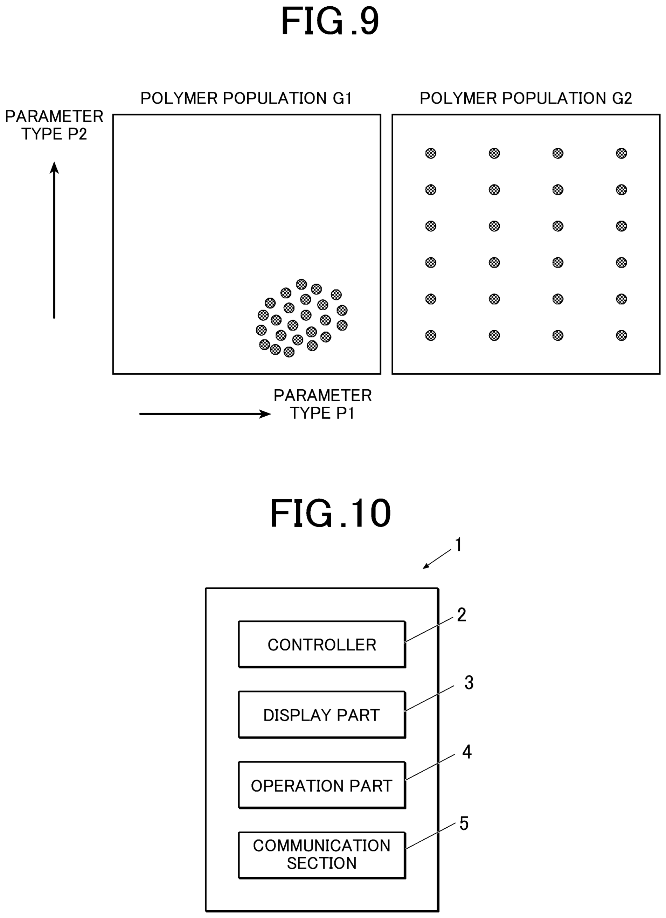

The degree of diversity in the polymer population is calculated using an evaluation function. FIG. 9 is a diagram showing the configurations of polymers in the polymer populations G1 and G2 when the horizontal axis represents the parameter type P1 and the vertical axis represents the parameter type P2 different from P1. In the polymer population G1 with a low degree of diversity, the polymers are relatively dense, and the coverage is narrow for both the parameter types P1 and P2. On the other hand, in the highly diverse polymer population G2, the polymers are relatively dispersed, and the coverage range is wide for both the parameter types P1 and P2. That is, the distance between the polymers included in the polymer population in the parameter space is calculated, and the degree of diversity can be evaluated from the calculated distance.

The distance between two polymers can be calculated using functions such as cosine similarity, Jaccard similarity, TF-IDF (Term Frequency-Inverse Document Frequency), Euclidean distance, Manhattan distance, Hamming distance, Jaccard similarity, correlation coefficient, Pearson correlation coefficient, dynamic programming (such as the Smith Waterman method and the Needleman-Wunsch method), dynamic time warping, and graph calculation distance.

The distance between n (three or more) polymers can be calculated by, for example, repeatedly calculating the distance between two polymers by the above function and calculating the Gini coefficient. The Gini coefficient can take a value within a range of 0 to 1. The Gini coefficient means that the larger the value of the coefficient is, the larger the difference in data in the group is, and means that the degree of diversity is high in the present embodiment. The Gini coefficient is expressed by the following (Expression 1), where n is the number of polymers, µ is an average of parameters, and x is a parameter value for each polymer. For example, xᵢᵢ is a value of the parameter type P1, and xⱼ is a value of the parameter type P2.$Gini = \frac{{\sum }_{i = 1}^{n} {\sum }_{j = 1}^{n} \left|{x}_{i}-{x}_{j}\right|}{2 {n}^{2} μ}$

However, in the calculation based on the Gini coefficient, the number of calculations increases as the number of polymers increases. Therefore, when the number of polymers is large, the degree of diversity may be evaluated by the following method.

When the number of polymers is large, for example, the diversity degree H (Xᵢ) is calculated for each parameter type (parameter value Xᵢ), and the diversity degrees H (Xᵢ) of different parameter types are added together. The parameter type means, for example, each of the parameter types P1 and P2. Here, when the degree of similarity between parameter types is high, the calculated degree of diversity tends to be higher than the actual degree of diversity. Therefore, for example, the similarity I (xᵢ, xⱼ) between the parameters is preferably subtracted from the sum of the diversity degrees H (Xᵢ). At this time, an evaluation function S is expressed as follows (Expression 2)). Note that λ means a hyper parameter.$S = {\sum }_{i = 1}^{n} H \left({X}_{i}\right) - λ {\sum }_{i \neq j} I \left({X}_{i}, {X}_{j}\right)$

The diversity degree H (Xᵢ) can be calculated using, for example, the Shannon entropy, the Simpson Index, the entropy, the variance Var (Xᵢ), or the like. The similarity I (Xᵢ, Xⱼ) can be calculated using, for example, mutual information amount, Wasserstein distances, and the absolute values [Corr Xᵢ, Xⱼ)|) of the correlations Corr(X ᵢ, Xⱼ) between the parameter type corresponding to the parameter value Xᵢ and the parameter type corresponding to the parameter Xⱼ.

In addition, the degree of diversity may be evaluated by assuming an arbitrary distribution Q having diversity and obtaining the Kullback - Leibler divergence (KLD) as the distance between the distribution X by the polymer population and the distribution Q. At this time, a small value of KLD means that the degree of diversity is high, and conversely, a large value of KLD means that the degree of diversity is low. In order to make the value calculated from KLD large when the degree of diversity is high, a method of multiplying by-1 is simple.

The degree of diversity may be evaluated using a determinant det (X ^{T} X) of X ^{T} X. When the degree of diversity is high, the value of the determinant is also large.

In a case where each of the plurality of parameters is converted into a different diversity of evaluation functions and the evaluation functions are summed, the summation becomes complicated. Therefore, plurality of parameters may be converted into one diversity evaluation function. For example, when a deep learning model to which both the primary structure and the higher-order structure of an aperiodic polymer are input is used, the vector can be converted into a vector reflecting both the primary structure and the higher-order structure.

It is preferable to set the evaluation function such that the higher the degree of diversity, the larger the value thereof, for example, such as multiplying by-1 as in the above-described KLD.

### (3) Extraction step

From the evaluation results of the evaluation process S12, combinations of aperiodic polymers (polymer populations) that are equal to or more than a threshold value are extracted. The extracted polymer population is used as a library. One or a plurality of polymer populations having an evaluation result of the evaluation step S12 that is equal to or greater than a threshold value may be extracted and used as a library. Furthermore, a plurality of polymer populations having an evaluation result of the evaluation step S12 that is equal to or greater than a threshold value may be extracted, and the plurality of extracted polymer populations may be combined into one library.

In the present embodiment, the evaluation step S12 and the extraction step S13 may be repeated. For example, first, a large amount of an aperiodic polymer is produced in the production process S11. In the first evaluation step S12, a plurality of small-scale polymer populations in which the number of aperiodic polymers is relatively small are formed, and the degree of diversity is evaluated for each of the small-scale polymer populations using an evaluation function. In the first extraction step S13, a plurality of polymer populations whose evaluation results are greater than or equal to the threshold value are extracted, and a group (small) of sub-libraries having diversity is formed by the plurality of extracted polymer populations. In the second evaluation step S12, the degree of diversity is evaluated using an evaluation function for each of the plurality of formed groups (small) of sub-libraries. In the second extraction step S13, a plurality of sub-library groups (small) whose evaluation results are equal to or greater than the threshold value are extracted, and a sub-library group (large) having diversity is formed by the plurality of extracted sub-library groups (small). A group (large) of sub-libraries may be used as the library, or a series of steps may be repeated to further comb through the groups of sub-libraries to finally obtain a library containing a certain number of aperiodic polymers. In addition, the final library may be not only a collection of groups of sub-libraries, but also a combination of a group of sub-libraries and one type of aperiodic polymer.

For example, a polymer population is formed with a relatively large number of aperiodic polymers at once by a method in which the evaluation step S12 and the extraction step S13 are not repeated. Therefore, the polymer population may include some aperiodic polymers having similar properties. Furthermore, the calculation step in the evaluation step S12 is complicated. On the other hand, by a method of repeating the evaluation step S12 and the extraction step S13, a polymer population is formed with a relatively small number of aperiodic polymers, and the number of aperiodic polymers is increased each time a group of sub-libraries is formed. Therefore, the polymer population and the group of sub-libraries are unlikely to contain aperiodic polymers with similar properties. In addition, although it is necessary to repeatedly perform the evaluation process S12, since the calculation process in each evaluation process S12 is relatively simple, it is possible to simplify the calculation process in the entire evaluation process S12 and improve the search efficiency.

In the series of steps from the generation step S11 to the extraction step S13, the conditions may be appropriately adjusted as described below.

For example, in a case where an aperiodic polymer which reacts with a target A but does not react with a target B is searched for, when an aperiodic polymer which is already known or presumed to react with target B is added as a candidate, the efficiency of the search deteriorates. Therefore, at the time of each step, before and after the step, and between the steps may be arbitrarily adjusted so as not to select an aperiodic polymer that is known or predicted to react with the target B.

For example, in the generation process S11, control may be performed so as not to generate an aperiodic polymer known or predicted to react with the target B. Furthermore, even if the aperiodic polymer is produced, the aperiodic polymer may be removed.

Furthermore, in the evaluation step S12, the evaluation function of an aperiodic polymer that is known or predicted to respond to the target B may be set to 0 or may be multiplied by a negative value. Depending on the number of aperiodic polymers known or predicted to respond to target B, a penalty term may be calculated and added. In calculating the degree of diversity, molecular space ranges known or predicted to respond to target B may be excluded. In calculating the degree of diversity, the diversity of aperiodic polymers known or predicted to respond to the target B may not be considered in the calculation, or may be given a penalty for selection in the subsequent extraction step S13.

Further, in the extraction step S13, an aperiodic polymer which is known or predicted to react with the target B may not be selected.

The adjustment in each of these steps may be arbitrarily performed without being limited to the case where the antibody reacts with the target A but does not react with the target B. For example, in searching for an aperiodic polymer that reacts with the target A, when there is an aperiodic polymer that is known or predicted to react with the target A, adjustment may be similarly performed during each step, before and after the step, and between the steps so as to positively select the aperiodic polymer. Furthermore, in a case where there are conditions that are to be avoided or are desirable in addition to a reaction, each step, before and after a step, and between steps may be similarly adjusted in accordance with these conditions. The conditions include, for example, molecular structure conditions (ease of attachment of a linker and the like), physicochemical conditions (ease of dissolution, low free energy, high stability of the most stable structure and the like), biological conditions (influence on the human body, ease of metabolism and the like), pharmacological conditions, information engineering conditions (only a small amount of calculation memory is required because the length of the aperiodic polymer is short and information is easily compressed, and the like), mathematical conditions and the like.

In addition, in a series of steps from the generation process S11 to the extraction process S13, comprehensiveness may be considered. The term "comprehensiveness" herein refers to a degree of how many of a plurality of aperiodic polymers can cover a predetermined range. As shown in FIG. 9, the coverage of the polymer population G2 is superior to that of the polymer population G1, and a library produced using the polymer population G2 can be searched more efficiently. That is, by controlling the comprehensiveness, an aperiodic polymer having desired properties can be efficiently searched for.

In addition, in a series of steps from the generation step S11 to the extraction step S13, densities or sparseness and denseness may be considered. The density or sparseness/denseness herein refers to a degree to which the plurality of aperiodic polymers are densely packed. As illustrated in Fig. 9, the densities are locally high in the polymer population G1 and are low in the other areas. On the other hand, in the polymer population G2, the density is homogeneous because of being evenly positioned in the whole. By controlling the density or the sparseness and denseness in this way, it is possible to efficiently search for an aperiodic polymer having desired properties.

### (4) Solid -phasing step

In an actual experiment, the library formed in extraction step S13 may be immobilized. First, an actual product of each aperiodic polymer extracted in the extraction step S13 is obtained. The method for obtaining the same is not particularly limited, and the same may be obtained in the form of a polymer, or may be obtained by synthesizing monomers. For the purpose of determining which polymer has reacted with a target, it is desirable that the polymer is immobilized. The aperiodic polymer may be synthesized and then solid-phased, or may be solid-phase-synthesized. A group of aperiodic polymers serves as a library.

For example, in the SELEX method, since a library in a free state in a solution is used, the type of the aperiodic polymer to be brought into contact with the target may be different for each experiment. On the other hand, in the immobilized library, the type and the position of the aperiodic polymer can be designated, and the same type of aperiodic polymer can be brought into contact for each experiment. Thus, the accuracy of the experiment can be improved by performing the experiment a plurality of times.

In the present embodiment, the term "solid phase" refers to a form in which an aperiodic polymer is covalently bonded to an object. It is important that the object and the aperiodic polymer are covalent bond. For example, the aperiodic polymer may be covalently bonded to the object as a result of being synthesized on the object, or a separately synthesized aperiodic polymer may be covalently bonded to the object. In a case where an object and an aperiodic polymer are covalent bond, the aperiodic polymer may be unlikely to form a higher-order structure or to bind to a target. Therefore, some spacer may be interposed between the object and the aperiodic polymer. Examples of the spacer include polyethylene glycol, tetraethylene glycol, ethylene glycol, and C₆H₁₄, C₁₂H₂₆.

The object on which the aperiodic polymer is solid-phased is not particularly limited. Examples of the object to be immobilized include a column, a film, a substrate, and a particle. Of these, the object to be solid-phased is preferably a substrate from the viewpoints of easy control of the solid-phase position of the aperiodic polymer and easy discrimination. Examples of the material of the object include glass, silica, and polyester. These may be modified in any way, from the viewpoints of ease of solid phase formation, storage stability, and the like.

### 3. Library production apparatus

The production apparatus of the present embodiment is an apparatus for producing the above-described library. FIG. 10 is a block diagram showing a schematic configuration of a library production apparatus in the present embodiment. The library production apparatus 1 includes a controller 2 (hardware processor), a display part 3, an operation part 4, and a communication section 5.

The controller 2 includes a CPU, a RAM, a memory, and the like. The CPU is a hardware processor that reads various programs stored in the memory, loads the programs to the RAM, and executes various types of processing according to the loaded programs to control the operation of the components of the library production apparatus 1. The various programs include a program for producing the above-described library.

In the present embodiment, the controller 2 combines at least two or more types of monomers selected from the monomer group to produce a plurality of types of aperiodic polymers. At this time, the controller 2 functions as a generation section. The controller 2 evaluates the diversity in the combination of the generated plurality of types of aperiodic polymers using an evaluation function. At this time, the controller 2 functions as an evaluation section. The controller 2 extracts a combination of aperiodic polymers whose evaluation result is equal to or greater than a threshold value. At this time, the controller 2 functions as an extraction section. Thus, a library having diversity in plurality of parameters can be produced.

The display part 3 and the operation part 4 include, for example, a liquid crystal display (LCD) with a touch screen. The display part 3 displays various operation screens, data, diversity evaluation results, aperiodic polymer extraction results, and the like in accordance with signals input from the controller 2. The operation part 4 includes various types of operation keys such as a numeric keypad and a start key, accepts various input operation by a user, and outputs a signal to the controller 2.

The communication section 5 transmits and receives various signals or data to and from an external device based on the communication control of the controller 2.

The library production apparatus may further include an apparatus for synthesizing an aperiodic polymer, an apparatus for immobilizing an aperiodic polymer, and the like, or may be externally connected to these apparatuses.

### 4. program

The program of the present embodiment is a computer program for producing the above-described library. In the present embodiment, the program combines at least two or more types of monomers selected from the group of monomers to produce a plurality of types of aperiodic polymers. The program evaluates the diversity of combinations of the generated plruality of types of aperiodic polymers using an evaluation function. The program extracts combinations of aperiodic polymers for which the evaluation result is greater than or equal to a threshold value. Thus, a library having diversity in plurality of parameters can be produced.

### [example]

Hereinafter, the present disclosure will be specifically described with reference to examples, but the present disclosure is not limited thereto. In the example, "part(s)" or "%" means "part(s) by mass" or "% by mass" unless otherwise specified.

In the following examples, operations were performed at room temperature (25°C) unless otherwise specified.

### <comparative example 1 >

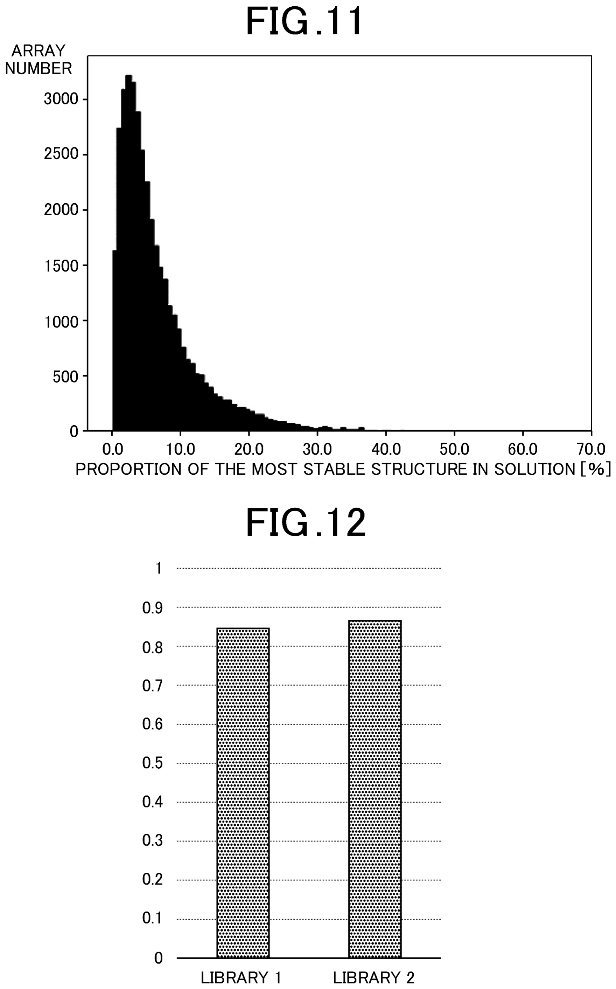

40000 types of random DNA primary structures were generated. The proportion of each DNA having the most stable higher-order structure was calculated by the ViennaRNA package, and the most frequent value was obtained at about 5% as in the histogram illustrated in Fig. 11. That is, it is found that in order to generate an aggregate of DNA having diversity, the primary structure needs to be generated so as to have a specific higher-order structure while being random. In addition, from this, it is considered that, in the SELEX method in which a library is prepared by generating a random primary structure, although the primary structure has randomness, the number of aperiodic polymers having a higher-order structure which actually reacts with a target is extremely small, and as a result, the diversity of the library is low.

### < example 1 >

A DNA library having eight patterns of higher-order structures shown in FIG. 7, i.e., having diversity in higher-order structures, was prepared, and the diversity was evaluated.

First, 10,000 primary structures of DNA of 20 to 60 bases were randomly generated using adenine, guanine, cytosine, and thymine as monomers.

### (primary structure parameters)

200000 kinds of primary structures of DNA which were separately generated and whose primary structures were random were encoded and learned by a variational autoencoder (VAE) model having a long short term memory (LSTM) in a feature extraction layer. The VAE model is a generative model that has learned random primary structures and can convert various primary structures into vectors that follow a normal distribution. Next, the generated 10,000 primary structures of the DNA whose primary structures are random were similarly input to the VAE model, and the converted vectors were used as parameters of the primary structures. When the primary structure parameter was defined as X, det (X ^{T} X) was calculated.

### (higher-order structure parameter)

For the higher-order structure parameter, the higher-order structure of each DNA was predicted by the ViennaRNA package, and as a result, a qualitative parameter indicating which of eight patterns the higher-order structure corresponds to, to be specific, Shannon entropy was calculated from the probabilities of the eight patterns of the higher-order structures.

### (diversity evaluation)

The calculation values of the primary structure parameters and the calculation values of the higher-order structure parameters were summed, and used as an index for evaluating the degree of diversity.

From the generated 10,000 random DNA primary structures, 100 were selected as a polymer population, and the diversity in the polymer population was evaluated. The diversity was evaluated again by changing the combination of the DNA primary structures forming the polymer population. This was repeated a total of 100 times, and the polymer population of the combination having the highest numerical value of the diversity evaluation index was designated as library 2.

For comparison, a library obtained by randomly selecting 100 patterns from the generated 10,000 patterns of random primary DNA structures was defined as library 1. The degree of diversity in Library 1 and Library 2 was evaluated.

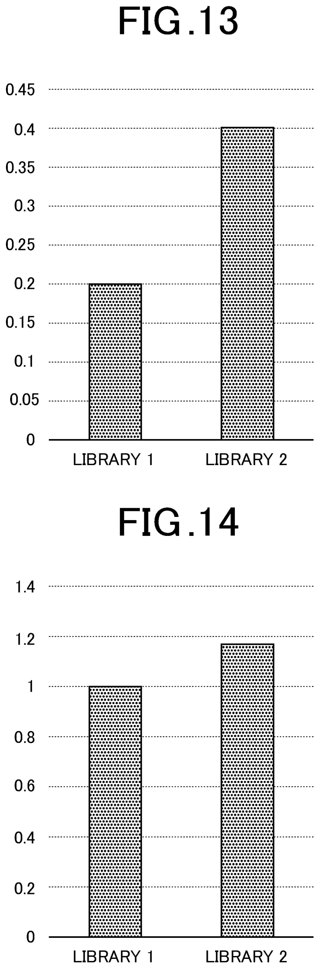

Fig. 12 is a graph of calculation values of primary structure parameters (indices for evaluating diversity of primary structures), and Fig. 13 is a graph of calculation values of higher-order structure parameters (indices for evaluating diversity of higher-order structures). Fig. 14 is a graph which shows the sum of the calculation values of primary structure parameters and the calculation values of higher-order structure parameters (diversity evaluation index). The value of the library 1 in the graph of FIG. 14 is set to 1, and the values of the other graphs are relatively represented, and the total value of the value of the library 1 in the graph of FIG. 12 and the value of the library 1 in the graph of FIG. 13 is 1.

As shown in FIGS. 12 to 14, in the library 2, the degree of diversity in the primary structure is maintained at the same level as in the library 1, and the degree of diversity in the higher-order structure is improved as compared with the library 1. In addition, as a result, it is found that the degree of total diversity is improved in the library 2 as compared with the library 1.

### < [Example 2] >

The library 1 and the library 2 produced in example 1 were asked to Agilent Technologies Inc. to be synthesized on a substrate, thereby producing a DNA microarray. The microarray was immersed in physiological saline containing 2% bovine serum albumin (BSA) to perform blocking. The blocked microarray was brought into contact with physiological saline containing immunoglobulin E (IgE) at 100µ g/mL, and washed with physiological saline.

The microarray was brought into contact with physiological saline containing a primary antibody against IgE at 20µ g/mL, and then washed with physiological saline. Next, the microarray was brought into contact with physiological saline containing a secondary antibody against IgE at 20µ g/mL, and then washed with physiological saline. Note that the secondary antibody against IgE was modified with a fluorescent molecule. Thereafter, the microarray was dried and photographed with a microarray scanner "SureScan (R)" (manufactured by Agilent Technologies), and the fluorescence of each spot of the microarray was quantified.

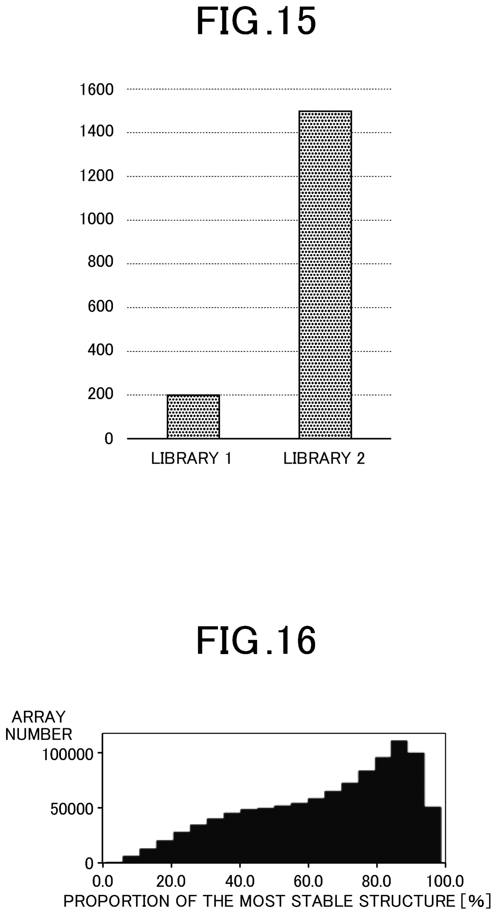

It can be determined that, in the microarray, a DNA having a high binding strength to IgE is immobilized on a spot having a high fluorescence intensity, whereas a DNA having a low binding strength to IgE is immobilized on a spot having a low fluorescence intensity. Fig. 15 is a graph illustrating the maximum intensity of fluorescence in the library 1 and the library 2. The maximum intensity is expressed as a relative value. Library 1 has diversity in only one type of parameter, while Library 2 has diversity in two types of parameters. As illustrated in Fig. 15, the library 2 has the highest luminance as compared with the library 1. That is, it can be seen that the library 2 actually contains aptamers having a stronger binding force by having diversity in two types of parameters, and aptamers having a stronger binding force can be easily searched for from the library 2.

### < Example 3 >

In example 1, library 2, the primary structure of DNA was generated so as to have eight patterns of higher-order structures, but library 3 was prepared in the same manner as library 2, except that the primary structure was generated so as to have a specific higher-order structure among the eight patterns.

A higher-order structure corresponding to any of the eight patterns was assigned to each of the ten thousand primary structures generated in example 1, and the primary structure was mutated so as to have the higher-order structure. The following examples are sequences having only one stem-loop, and represent, using the letters A, T, G, and C, base types arranged in the 5' to 3' direction from the left. Furthermore, the symbol .() is used to represent a higher-order structure in the dot-bracket format. Here, when it is assumed that aand T or G and C bind to each other by Watson -Crick hydrogen bonding to form a higher-order structure, there arises a contradiction that the fifth A and the 11th A from the 5'-side hydrogen-bond to each other, and therefore the fifth A is mutated to T.

GCCTATCCCAAACCCCCCCCCCCC
...(((…)))………..
↓
GCCTTTCCCAAACCCCCCCCCCCC
...(((…)))………..

The mutations were repeated in this way to generate an energetically stable primary structure. At this time, for a pattern having only one stem-loop, the proportion of each DNA taking the most stable higher-order structure by the ViennaRNA package was calculated. As indicated by the histogram in Fig. 16, it was confirmed that the proportion of assuming the most stable higher-order structure was improved as compared with Fig. 11 of comparative example 1.

Next, a VAE model using both the primary structure and the secondary structure of the generated DNA as inputs was learned. Again, the primary structure and the secondary structure of the DNA were input to the encoder and converted into latent space vector. The vectors of the respective DNAs were combined to create a matrix X, det (X ^{T} X) was calculated to calculate an evaluation index of diversity, and a polymer population of a combination providing the highest evaluation index of diversity was selected as the library 3.

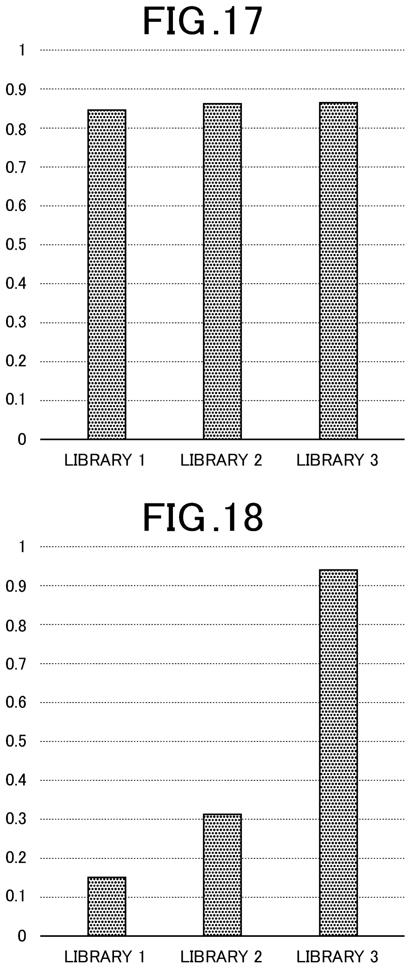

As illustrated in Figs. 17 to 19, it is found that the degree of diversity is further improved in the library 3 than in the library 2. Note that the value of the library 1 in the graph of Fig. 19 is set to 1, and the values of the other graphs are expressed relatively. From FIG. 18, since the degree of diversity in the higher-order structure is particularly improved, it is found that the degree of diversity in the higher-order structure can be easily improved while maintaining the degree of diversity in the primary structure by generating a primary structure in which a desired higher-order structure is energetically stable.

According to the above embodiment, the efficiency of searching for an aperiodic polymer that reacts with a target can be improved.

An aperiodic polymer that reacts with a target can be searched for using a library. The library contains a large number of candidate aperiodic polymers. In the library produced by the production method of the present embodiment, the degree of diversity in a plurality of parameters is evaluated using an evaluation function, and the evaluation result is equal to or greater than a threshold value, that is, the library has diversity. Therefore, there is a high possibility that the library contains an aperiodic polymer that reacts with a target, and an aperiodic polymer that reacts with a target can be efficiently searched.

Furthermore, according to the means of the present disclosure, the diversity of the library can be improved, and furthermore, a library that covers the parameters that the aperiodic polymer can exert can be constructed. Since the library has high coverage, there is a high possibility that an aperiodic polymer having parameters close to desired properties is included in the library, and thus there is a high probability that an aperiodic polymer can be searched for.

Although several embodiments of the present invention have been described, the scope of the present invention is not limited to the above-described embodiments, but encompasses the scope of the invention described in the claims and equivalents thereof.

Although embodiments of the present invention have been described and illustrated in detail, the disclosed embodiments are made for purposes of illustration and example only and not limitation. The scope of the present invention should be interpreted by terms of the appended claims.

## Claims

1. A method for producing a library that includes a plurality of types of aperiodic polymers, each of the aperiodic polymers being an aperiodic polymer, and the method comprising:
generating that is generating a plurality of types of the aperiodic polymers by combining two or more types of monomers selected from a monomer group;
evaluating that is evaluating, by using an evaluation function, diversity in a combination of the generated plurality of types of the aperiodic polymers; and
extracting that is extracting a combination of the aperiodic polymers for which an evaluation result is equal to or greater than a threshold value, wherein
the evaluation function is a function for evaluating diversity based on two or more types of parameters.

2. The method according to claim 1, wherein the parameters include two or more types of parameters belonging to a group including a primary structure, a higher-order structure, a type of an element, a number of atoms, a coordinate of the atoms, a position of the atoms, a free energy of a most stable structure, a proportion of the most stable structure, a melting temperature, a length, a polarizability, a polarity, a reactivity, an orientation, a solubility, a similarity between aperiodic polymers, predictions thereof and conversions thereof, in the aperiodic polymers.

3. The method according to claim 2, wherein the parameters include one type or more belonging to a group including the primary structure, the higher-order structure, a prediction of the primary structure, a conversion of the primary structure, a prediction of the higher-order structure, and a conversion of the higher-order structure.

4. The method according to claim 1 or 2, wherein in the generating, a primary structure is generated such that the aperiodic polymer has a predetermined higher-order structure.

5. The method according to claim 4, wherein in the generating, the primary structure is generated such that the aperiodic polymer is most stable in terms of energy when the aperiodic polymer has the predetermined higher-order structure.

6. The method according to claim 1 or 2, wherein the evaluating and the extracting are repeated a plurality of times.

7. The method according to claim 1 or 2, wherein a number of monomers included in one molecule of the aperiodic polymer is within a range of 10 to 100.

8. The method according to claim 1 or 2, wherein the aperiodic polymer is a nucleic acid.

9. A library production apparatus for producing a library that includes a plurality of types of aperiodic polymers, the apparatus comprising:
a generation section (2) that generates a plurality of types of the aperiodic polymers by combining two or more types of monomers selected from a monomer group;
an evaluation section (2) that evaluates, by using an evaluation function, diversity in a combination of the generated plurality of types of the aperiodic polymers; and
an extraction section (2) that extracts a combination of the aperiodic polymers for which an evaluation result is equal to or greater than a threshold value, wherein
the evaluation function is a function for evaluating diversity based on two or more types of parameters.

10. A program for a computer in a library production apparatus for producing a library that includes a plurality of types of aperiodic polymers, the program causing the computer to execute:
generating that is generating a plurality of types of the aperiodic polymers by combining two or more types of monomers selected from a monomer group;
evaluating that is evaluating, by using an evaluation function, diversity in a combination of the generated plurality of types of the aperiodic polymers; and
extracting that is extracting a combination of the aperiodic polymers for which an evaluation result is equal to or greater than a threshold value, wherein
the evaluation function is a function for evaluating diversity based on two or more types of parameters.

11. A method for searching for an aperiodic polymer that reacts with a target by using a library, the method comprising:
producing the library by using the method according to claim 1 or 2;
contacting the produced library with the target; and
collecting the aperiodic polymer reacted with the target.
